# EUROPEAN PATENT SPECIFICATION

(11) **EP 2 749 266 B1**
(45) Date of publication and mention of the grant of the patent: **29.08.2018**
(21) Application number: 12008626.9
(22) Date of filing: 27.12.2012
(51) Int. Cl.: A61K 47/10, A61K 47/14, A61K 31/592, A61K 9/00

(54) **Stable injectable pharmaceutical composition of vitamin D receptor agonist and process for preparation thereof**
Stabile injizierbare pharmazeutische Zusammensetzung eines Vitamin-D-Rezeptoragonisten und Prozess zur Herstellung davon
Composition pharmaceutique injectable stable d'agoniste du récepteur de la vitamine D et son procédé de préparation

(43) Date of publication of application: 02.07.2014
(73) Proprietor: Pharmathen S.A., Pallini Attikis 15351 (GR)
(72) Inventor: Koutris, Efthimios, P.C. 15351 Pallini Attikis (GR); Karavas, Evangelos, P.C. 15351 Pallini Attikis (GR); Koutri, Ioanna, P.C. 15351 Pallini Attikis (GR); Abatzis, Morfis, P.C. 15351 Pallini Attikis (GR); Samara, Vasiliki, P.C. 15351 Pallini Attikis (GR); Iliopoulou, Athina, P.C. 15351 Pallini Attikis (GR)
(74) Representative: Brown, Andrew Stephen

(56) References cited:
- EP-A1- 0 390 930
- WO-A2-00/61112
- US-A1- 2010 075 933

## Description

### TECHNICAL FIELD OF THE INVENTION

The present invention relates to a stable injectable pharmaceutical composition comprising a vitamin D receptor agonist such as paricalcitol and a process for manufacturing such composition.

### BACKGROUND OF THE INVENTION

Vitamins are organic chemical compounds (or related set of compounds) that cannot be synthesized in sufficient quantities by an organism and must be obtained from diet. In particular, Vitamin D is found in many dietary sources such as fish, eggs, fortified milk, and cod liver oil. The sun also contributes significantly to the daily production of vitamin D, and as little as 10 minutes of exposure is thought to be enough to prevent deficiencies. The term "vitamin D" refers to several different forms of this vitamin. Two forms are important in humans: ergocalciferol (vitamin D₂) and cholecalciferol (vitamin D₃). Vitamin D₂ is synthesized by plants. Vitamin D₃ is synthesized by humans in the skin when it is exposed to ultraviolet-B (UVB) rays from sunlight. Foods may be fortified with vitamin D₂ or D₃.

Although the synthesis of Vitamin D₃ occurs naturally in the skin with adequate sunlight exposure, Vitamin D₃ is not active and needs to be converted to 25(OH) D₃ in the liver. From the liver, 25(OH) D₃ is transported to the kidney and hydroxylated by 25-hydroxyvitamin D₃1-hydroxylase to form the active hormone calcitriol. Said active form binds to vitamin D receptors (VDRs) that are present in the parathyroid gland, intestine, kidney, and bone to maintain parathyroid function and calcium and phosphorus homeostasis and to VDRs found in many other tissues, including prostate, endothelium and immune cells. VDR activation is essential for the proper formation and maintenance of normal bone. In the diseased kidney, the activation of Vitamin D is diminished, resulting in a rise of parathyroid hormone, subsequently leading to secondary parathyroidism and disturbances in the calcium and phosphorus homeostasis.

Vitamin D deficiency leads to numerous diseases and physical ailments. Rickets and osteomalacia are classic vitamin D deficiency diseases. In children, vitamin D deficiency causes rickets, which results in skeletal deformities. In adults, vitamin D deficiency can lead to osteomalacia, which results in muscular weakness in addition to weak bones.

Paricalcitol, also called 19-nor-1,25-(OH)₂-vitamin D₂ or 19-nor-1,25-dihydroxyvitamin D₂, is a synthetic, biologically active Vitamin D analog of calcitriol with modifications to the side chain (D2) and the A (19-nor) ring allowing for selective vitamin D receptor activation. Paricalcitol reduces parathyroid hormone levels by inhibiting parathyroid proliferation. Thus, it is used for the prevention and treatment of secondary hyperparathyroidismin people with chronic renal failure.

The chemical name of paricalcitol is 19-nor-1α,3β,25-trihydroxy-9,10-secoergosta-5(Z),7(E), 22(E)-triene. The molecular formula is C₂₇H₄₄O₃, which corresponds to a molecular weight of 416.64. It is a white to almost white powder which is soluble in alcohol and insoluble in water.

EP-B-1073467 relates to a sterilized, self-preserved, aqueous composition for parenteral administration comprising a Vitamin D compound, water and an organic solvent selected from aliphatic alcohols of from 1 to 5 carbons and glycol derivatives, such as propylene glycol The formulation of the original paricalcitol injectable product Zemplar® falls inside the scope of this patent and comprises ethanol (20% w/v), propylene glycol and water.

EP-B-0390930 refers to an aqueous preparation of active form of vitamin D3 wherein the active form of vitamin D3 is solubilized with a nonionic surface active agent and stabilized with a combination of at least one chelating agent selected from citric acid, tartaric acid and their metal salts with an antioxidant.

EP-B-0100459 discloses an aqueous liquid containing a fat soluble substance such asVitamin D, lecithin and at least one alcohol (ethanol or isopropanol) in certain ratios.

Although each of the patents above represents an attempt to overcome the stability problems associated with parenteral compositions of paricalcitol, there still remains the need in the art for a patient-friendly injectable composition with improved stability.

### SUMMARY OF THE INVENTION

It is, therefore, an object of the present invention to provide a thermodynamically stable and efficient soluble product comprising paricalcitol suitable for intravenous administration.

A major object of the present invention is to provide a liquid drug preparation of paricalcitol for intravenous administration, which overcomes the major adverse effects associated with such route of administration. The most common side effects related to intravenous administration are haemolysis, precipitation, irritation, sensitization, phlebitis and pain at the site of injection.

It is another object of the present invention to provide a safe and efficacious injectable preparation containing paricalcitol. The intravenous solution of the present invention is subjected to sterilization processes (Filtration-Autoclaving) according to the Pharmacopoeia requirements (European Pharmacopoeia 5.0; 01/2005:50101 and 01/2005:50102). Heat sterilization does not affect the finished dosage form of the present invention.

The present invention aims at developing a formulation that not only matches the physical and chemical attributes of the reference product but also overcomes the disadvantages associated with the prior art compositions.

A further approach of the present invention is to provide a fast, simple and cost-effective process for the preparation of a stable injectable pharmaceutical formulation comprising paricalcitol.

In accordance with the above objects of the present invention we present a process for the preparation of a stable pharmaceutical composition comprising paricalcitol for intravenous administration comprising the following stages:
- dissolving paricalcitol or an ester, isomer, solvate, hydrate or polymorph thereof in a solution of macrogol 15 hydroxystearate with an alcohol selected from ethanol, propanol, butanol or benzyl alcohol,
- adjusting the volume with water for injection and, optionally,
- sterilizing through filtration and/or autoclaving.

Preferred suitable co-solvents include alcohols selected from ethanol, propanol (preferably iso-propanol), butanol and benzyl alcohol. Preferably the alcohol is with water. A preferred alcohol is ethanol. The co-solvent should aid the solubilisation of paricalcitol and be suitable for use in injectable formulations for humans. The co-solvent may also have one or more secondary function associated with that excipient, such as acting as a preservative. However, the primary consideration for the addition of a co-solvent to a composition of the invention is that it is reauired to aid the dissolving of the Vitamin D compound, in particular paricalcitol.

Other objects and advantages of the present invention will become apparent to those skilled in the art in view of the following detailed description.

### BRIEF DESCRIPTION OF THE DRAWINGS

Fig. 1 shows the stability results of % difference of assay up to 6 months of a composition with Solutol® HS 15 not autoclaved.
Fig. 2 shows the stability results of % difference of assay up to 6 months of a composition with Solutol® HS 15 autoclaved.

### DETAILED DESCRIPTION OF THE INVENTION

For the purposes of the present invention, a pharmaceutical composition comprising an active ingredient is considered to be "stable" if said ingredient degrades less or more slowly than it does on its own and/or in known pharmaceutical compositions. Acceptable levels of stability are where the composition is stored at 40°C for 3 months and less than 5%wt of the active ingredient has degraded.

The present invention is prepared according to the co-solvency approach. Co-solvents are defined as water-miscible organic solvents that are used in liquid drug formulations to increase the solubility of poorly water-soluble substances such as paricalcitol and to enhance the chemical stability of the drug. Examples of common co-solvents include salts, alcohols, polyols and carbohydrates. Most preferably alcohols, such as methanol, ethanol and isopropyl alcohol are used as co-solvents with water.

Compared with other routes of administration, the intravenous route is the fastest way to deliver medications to the site of interest. It is, without doubt, the most efficient and accurate route. However, it is associated with adverse effects that may be harmful or cause discomfort to the patient. The non-physiologic osmolality, viscosity and pH of drug solutions are mechanisms of inflammation and histologic changes in vessels resulting in uncomfortable sensations for patients under such treatment. The present invention aims at reducing the incidence of side effects when paricalcitol is administered intravenously.

Pain and irritation at the site of injection may be related to the formulation vehicle, particular those that contain high fractions of co-solvents or those that have high osmolalities. There have been numerous methods published relating to the assessment of pain. These methods can be characterized as reflexive (tail-flick, paw-lick, hot plate or pinch test), conscious escape (flinch-jump test), prolonged protective activity (fleeting/fighting) and retreat/withdrawal responses. Suitable test are described in Neuroscience: Bannon, A. W. and Malmberg, A. B. 2007; Models of Nociception: Hot plate, Tail-flick and Formalin tests in rodents. Current Protocols in Neuroscience; 41:8.9.1-8.9.16.

Haemolysis is the excessive breakdown of red blood cells. It can result from osmotic action or from the effect of either the drug or the formulation components on cell membranes. Although haemolysis is not strictly proportional to solution tonicity, it is definitely a function of vehicle composition. Drug solutions with high osmolality may cause haemolysis. The loss of integrity of red blood cell membranes releases the cellular contents into the plasma. If the release of free haemoglobin into the circulation is more than the body can clear, a number of symptoms can result, including fever, chills, abdominal and back pain, shortness of breath, prostration, and shock. High plasma concentrations of haemoglobin can lead to plaque formation and the clogging of renal tubules, thereby affecting kidney function. Haemolysis may also produce congestion in the reticuloendothelial cells of the spleen and liver causing splenomegaly and jaundice, respectively. When a formulation is injected, red blood cells are displaced from the centre by the formulation itself. Only those cells that are in contact with a sufficiently high concentration of formulation are prone to haemolysis. The maximum tolerable concentration that will not produce haemolysis is dependent upon the composition of the formulation.

Propylene glycol is a widely used vehicle for water-insoluble drugs. Also propylene glycol is found in the original formulation of paricalcitol the brand Zemplar®. It is used as a solvent not only in injectable but also in oral and topical formulations. However, the injection of vitamin D compounds formulated with this solvent often results in pain, haemolysis and thrombophlebitis. The pain may be reduced by premedication with local anaesthetics or opioids. The premedication, however, does not abolish the underlying mechanism of tissue damage. After extensive studies, it was surprisingly found that the use of a non-ionic solubilizer and particularly one with low osmolality effectively reduced the incidence of side effects.

The amount of the non-ionic solubilizer of the intravenous composition of the present invention varies between 0.1% w/v to 5% w/v of the total volume. At least 0.2% w/v, 0.3% w/v, 0.4% w/v, at least 0.45% w/v. Preferably less than 5% w/v, 4.5% w/v, 4% w/v, 3.5% w/v and 3% w/v.

Non-ionic solubilizers that can be used as vehicles for parenteral dosage forms according to the present invention include, but are not limited to one or more of the following monoesters of polyoxyethylenesorbitan fatty acids (Tween®, Liposorb®), monoesters of sorbitan fatty acids (Span®), polyethylene glycol 15-hydroxystearate (Solutol HS 15®), polyethylene glycol esters of fatty acids (Kessco®, Emerest®), polyoxyethylene glycol esters (Emulphor®), polyethoxylated castor oils (Cremophor®, Simusol®), polyglycerol esters of fatty acids (Caprol®, Nikkol®), polyethylene glycol ethers (Volpe®, Brij®), poloxamers (Lutrol®, Pluronic®), polyoxyethylene phenyl ethers (Triton®, Igepal®), or mixtures thereof.

Specifically preferred non-ionic solubilizers are selected from one of more of the following: macrogol (25)-cetostearyl ether, polyethylene glycol 1100 mono(hexadecyl/octadecyl) ether poloxamer 188 poly(ethylene glycol)-block-poly(propyleneglycol)-block-poly(ethylene glycol), poloxamer 407 poly(ethylene glycol)-block-poly(propylene glycol)-block-poly(ethylene glycol), macrogolglycerolricinoleate, PEG-35 castor oil,polyoxyl 35 hydrogenated castor oil, polyoxyl-35 castor oil, macrogol (15)-hydroxystearate, polyethylene glycol (15)-hydroxystearate, polyoxyethylated 12-hydroxystearic acid, macrogol 15 hydroxystearate, macrogolglycerolhydroxystearate PEG-40 castor oil, polyoxyl 40 hydrogenated castor oil, polyethylene glycol 300, poly(ethylene glycol)-block-poly(propylene glycol)-block-poly(ethylene glycol),cetyl alcohol, ceteth-2, ceteth-20, cetearylalcohol, PEG-40 hydrogenated castor oil, PEG-60 hydrogenated castor oil, lauryl alcohol, laureth-12 and the like, oleth-10 and the like, oleylalcohol, PPG-15 stearyl ether, stearylalcohol, steareth-2 and the like, cetearylalcohol, ceteareth-20, trideceth-12, glycerylstearateand PEG-100 stearate, cetearylalcohol and glycerylstearate and PEG-40 stearate and ceteareth-20, stearylstearate, PEG-20stearate, sorbitansesquioleate, sorbitanoleate, sorbitanpalmitate, sorbitanstearate, polysorbate85, sorbitantristearate, polysorbate 60, PEG-2 laurate SE, glycoldistearate, glycerylstearate, glycol stearate and stearamide AMP and Macrogol-glycerol hydroxystearate.

Most preferably the non-ionic solubilizer is Solutol HS 15®.

As indicative of the osmolality that can be reached with the use of a non-ionic solubilizer in comparison with propylene glycol, formulations comprising paricalcitol and propylene glycol or Solutol HS 15® were prepared. The results are presented in table 1 below.

**Table 1: Comparison of osmolalities between Solutol HS 15® and propylene glycol**

| **% concentration of the initial solution** | **Propylene Glycol** | **0.5% w/v Solutol HS 15®** |
|---|---|---|
| 10 | 827 | 305 |
| 12.5 | 1078 | 348 |
| 16.7 | 1524 | 477 |
| 20 | 1923 | 596 |
| 25 | 2675 | 787 |

Osmolality measurements are in milli-osmoles per kilogram. A preferred ranges of osmolality are less than 800, ideally less than 750, 700, 650 or 600, but more than 200, ideally more than 250 or 300,milli-osmoles per kilogram as measured by a membrane osmometer.

Macrogol 15 hydroxystearate (Solutol HS 15®) is a mixture of mainly monoesters and diesters of 12-hydroxystearic acid and macrogols obtained by the ethoxylation of 12-hydroxystearic acid. The number of moles of ethylene oxide reacted per mole of 12-hydroxystearic acid is 15. It contains about 30% free macrogols.

Solutol HS 15® has been monographed in the European Pharmacopoeia under the monograph name "Macrogol 15 Hydroxystearate" (European Pharmacopoeia 5.0; 01/2005:2052). Its chemical name is polyethylene glycol-15-hydroxystearate. Macrogol 15 hydroxystearate forms spherical micelles. The incorporation of water-insoluble drugs, such as paricalcitol, into the micelles is a favourable method to yield a sufficient and suitable drug solution. The solubilising capacity of Macrogol 15 hydroxystearate increases almost linearly with increasing concentration of the solubilising agent due to the formation of loose and porous spherical micelles with a low package density even at high concentrations. Similarly, tests have revealed that the viscosity increases with increasing amount of solubilizer, but the amount of solubilised paricalcitol does not have any additional influence on the kinematic viscosity. Macrogol 15 hydroxystearate meets the requirements of an effective solubilizer for parenteral use combining high solubilising capacity and low toxicity.

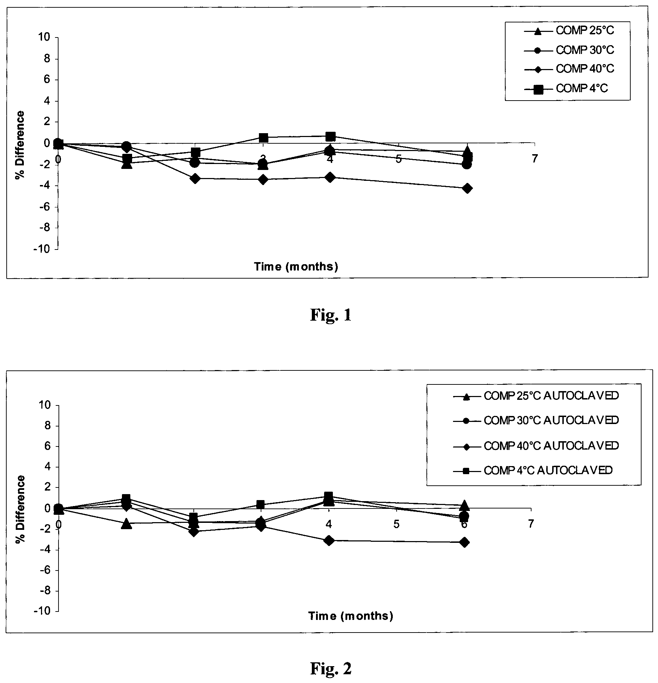

Another advantage of Macrogol 15 hydroxystearate unlike other solubilizers, is that is not affected by the sterilization process (Autoclaving/T=121°C). An assay up to 6 months proved that Macrogol 15 hydroxystearate was stable to heat sterilization (Fig. 1, 2). Tests have not revealed any changes of properties after sterilization indicating that no considerable hydrolyzation took place. Also the micelle diameter remained unchanged.

The only purely physical problem associated with the intravenous injection of drugs is phase separation, i.e., the formation of oil droplets or crystals of drug upon mixing of the formulation with blood. Drugs solubilized by the means of co-solvency can precipitate when diluted or injected into the bloodstream. When the formulation is diluted to the point at which the concentration of co-solvent is not sufficient to maintain the solubility of the drug, precipitation will occur. If a drug precipitates in the vein the potential for venous irritation, due not only to mechanical irritation but also to prolonged local drug exposure at the vein wall, increases. This painful condition is called phlebitis. Phlebitis can result from mechanical irritation, chemical irritation, or as a pharmacological response by the vein wall cells to the drug. If the precipitated material is crystalline, the particles can cause cellular abrasion as they move along the vein wall and local toxicity as well. Precipitation of solubilized drugs can also result in uneven or delayed bioavailability. In fact, the mere presence of a separate drug phase indicates a reduced concentration in the aqueous phase. If the second phase is rapidly re-dissolved there may be no loss in total efficacy. However, if it becomes embedded into or sorbed onto the vein cell wall and re-dissolution is gradual, either reduced or prolonged efficacy can result. Paricalcitol IV formulation with Solutol HS 15® after one month storage at low (4°C) and normal temperature (25°C) presented no precipitation of the vitamin D compound.

The injectable composition of the present invention comprising paricalcitol or an ester, isomer, solvate, hydrate or polymorph thereof, further comprises at least one co-solvent. Preferably the co-solvent is in an amount of less than 50% (v/v) and most preferably in an amount of less than 45% (v/v), less than 40% (v/v), less than 35% (v/v), less than 30% (v/v) or less than 25% (v/v). A preferred amount is 20% (v/v). Preferably at least one other solvent preferably water, in amount of more than 50% (v/v), more than 55% (v/v), more than 60% (v/v) and preferably more than 65% (v/v), and most preferably in an amount of more than 70%(v/v).

The present invention includes the vitamin D compound paricalcitol or a salt, isomer, solvate, ester, hydrate or polymorph thereof.

Surprisingly, it was found the parenteral composition according to the present invention is a self preserved formulation and possesses excellent physical and chemical stability. The composition of the present invention is free of any additional preservatives. No precipitation is observed and remains sterile without the presence of any additional preservative. Some common preservatives in wide use are selected from one or more of the following: propionic acid, nitrates and nitrites, benzyl alcohol, chlorbutanol, parabens, benzoic acid, chlorhexidine, benzalkonium chloride and imidurea.

The composition of the present invention is preferably ready to use but can be formulated as lyophilized powder which can reconstituted prior to administration. Said composition can be in a vial, an ampoule or a prefilled syringe but also can be provided as a kit comprising lyophilized powder in a vial and the reconstitution liquid in a separate vial.

The pharmaceutical composition according to the present invention may include pharmaceutically acceptable excipients such as chelating agents, preservatives, in accordance with claim 1 herein, surfactants, buffers, pH adjusting agents and other well-known excipients used for parenteral administration formulations.

Suitable surfactants may be selected form sodium lauryl sulphate, docusate sodium, butylparaben, polysorbate. Buffers or pH adjusting agents may be selected from hydrochloric acid, sodium hydrogen sulphate, sodium citrate dehydrate. Chelating agents may be selected from inorganic or organic phosphates, disodium edentate, fumaric acid.

### EXAMPLES:

### Example 1

**Table 3: Paricalcitol composition of example 1**

| **Ingredients** | **% content** |
|---|---|
| Paricalcitol | 0.0005 w/v |
| Solutol HS 15® | 0.5000 w/v |
| Ethanol absolute | 20.0000 v/v |
| Water for injection | q.s. |
| TOTAL | 100.00 v/v |

The paricalcitol intravenous solution of Table 3 is prepared according to the following manufacturing process:
- The appropriate quantity of paricalcitol is weighed;
- Solutol HS 15® is added into ethanol absolute and the resulting mixture is stirred until complete dissolution of the former;
- Paricalcitol is added into the above solution and the resulting mixture is stirred until complete dissolution of the active;
- The volume is adjusted with water for injection.

The resultant paricalcitol solution is filtered through 0.22µm filters and filled into glass ampoules or vials. The ampoules or vials of paricalcitol solution are sterilized in the autoclave at 121°C for at least 15 minute

Analytical procedures and tests were performed for paricalcitol IV product in order to prove that it meets the current technical specifications. The stability results for the impurities showed that the finished dosage form was not affected by heat sterilization (Autoclaving/T=121°C). The stability trial results are summarized in Tables 4 below.

**Table 4: Stability results for impurities up to 6 months (Product not autoclaved/autoclaved)**

| | **NOT AUTOCLAVED** | | | |
|---|---|---|---|---|
| | **25°C** | **30°C** | **40°C** | **4°C** |
| **3 months** | Imp A: ND | Imp A: ND | Imp A: ND | Imp A: ND |
| | Imp B: ND | Imp B: ND | Imp B: ND | Imp B: ND |
| | Imp C: 0.04 | Imp C: 0.05 | Imp C: 0.05 | Imp C: 0.07 |
| | Imp D: ND | Imp D: ND | Imp D: ND | Imp D: ND |
| | Imp E: 0.18 | Imp E: 0.23 | Imp E: ND | Imp E: 0.08 |
| | Imp F: ND | Imp F: 0.11 | Imp F: ND | Imp F: 0.21 |
| | Imp G: ND | Imp G: ND | Imp G: ND | Imp G: ND |
| | Imp H: ND | Imp H: ND | Imp H: ND | Imp H: ND |
| | Imp I: ND | Imp I: ND | Imp I: ND | Imp I: ND |
| | Total unknown: 0.28 | Total unknown: 0.67 | Total unknown: 1.1 | Total unknown: 0.23 |
| | **Total: 0.50** | **Total: 1.06** | **Total: 1.15** | **Total: 0.59** |
| **6 months** | Imp A: ND | Imp A: ND | Imp A: ND | Imp A: ND |
| | Imp B: 0.06 | Imp B: 0.07 | Imp B: 0.2 | Imp B: 0.06 |
| | Imp C: ND | Imp C: 0.11 | Imp C: 0.05 | Imp C: 0.03 |
| | Imp D: ND | Imp D: ND | Imp D: ND | Imp D: ND |
| | Imp E: ND | Imp E: ND | Imp E: ND | Imp E: 0.22 |
| | Imp F: ND | Imp F: ND | Imp F: ND | Imp F: ND |
| | Imp G: ND | Imp G: ND | Imp G: ND | Imp G: ND |
| | Imp H: ND | Imp H: ND | Imp H: ND | Imp H: ND |
| | Imp I: ND | Imp I: ND | Imp I: ND | Imp I: ND |
| | Total unknown: 0.94 | Total unknown: 1.61 | Total unknown: 1.71 | Total unknown: 0.89 |
| | **Total: 1.0** | **Total: 1.79** | **Total: 1.96** | **Total: 1.20** |

| | **AUTOCLAVED** | | | |
|---|---|---|---|---|
| **3 months** | Imp A: ND | Imp A: ND | Imp A: ND | Imp A: ND |
| | Imp B: ND | Imp B: ND | Imp B: ND | Imp B: ND |
| | Imp C: 0.03 | Imp C: ND | Imp C: ND | Imp C: ND |
| | Imp D: ND | Imp D: ND | Imp D: ND | Imp D: ND |
| | Imp E: 0.08 | Imp E: ND | Imp E: 0.19 | Imp E: ND |
| | Imp F: 0.16 | Imp F: ND | Imp F: ND | Imp F: 0.19 |
| | Imp G: ND | Imp G: ND | Imp G: ND | Imp G: ND |
| | Imp H: ND | Imp H: ND | Imp H: ND | Imp H: ND |
| | Imp I: ND | Imp I: ND | Imp I: ND | Imp I: ND |
| | Total unknown: 0.19 | Total unknown: 0.73 | Total unknown: 0.63 | Total unknown: 0.26 |
| | **Total: 0.46** | **Total: 0.73** | **Total: 0.82** | **Total: 0.45** |
| **6 months** | Imp A: ND | Imp A: ND | Imp A: ND | Imp A: |
| | Imp B: 0.07 | Imp B: ND | Imp B: ND | Imp B: |
| | Imp C: 0.1 | Imp C: ND | Imp C: ND | Imp C: 0.09 |
| | Imp D: ND | Imp D: ND | Imp D: ND | Imp D: |
| | Imp E: 0.25 | Imp E: ND | Imp E: ND | Imp E: 0.23 |
| | Imp F: ND | Imp F: ND | Imp F: ND | Imp F: 0.31 |
| | Imp G: ND | Imp G: ND | Imp G: ND | Imp G: |
| | Imp H: ND | Imp H: ND | Imp H: ND | Imp H: |
| | Imp I: ND | Imp I: ND | Imp I: ND | Imp I: |
| | Total unknown: 1.09 | Total unknown: 1.13 | Total unknown: 1.84 | Total unknown: 0.66 |
| | **Total: 1.51** | **Total: 1.13** | **Total: 1.84** | **Total: 1.30** |

## Claims

1. A pharmaceutical composition for parenteral administration comprising paricalcitol or an ester, isomer, solvate, hydrate or polymorph thereof, an alcohol selected from ethanol, propanol, butanol or benzyl alcohol and an effective amount of macrogol 15 hydroxystearate that stabilizes the composition both physically and chemically and provides low osmolality, wherein the composition is free of any additional preservative.

2. The pharmaceutical composition according to claim 1, wherein macrogol 15 hydroxystearate is in an amount between 0.1% w/v to 5% w/v of the total volume of the composition.

3. The pharmaceutical composition according to any preceding claim packaged in vial or ampoule or prefilled syringe.

4. A process for the preparation of a stable pharmaceutical composition comprising paricalcitol or an ester, isomer, solvate, hydrate or polymorph thereof for intravenous administration comprising the following stages:
- dissolving paricalcitol or an ester, isomer, solvate, hydrate or polymorph thereof in a solution of macrogol 15 hydroxystearate with an alcohol selected from ethanol, propanol, butanol or benzyl alcohol;
- adjusting the volume with water for injection and, optionally,
- sterilizing through filtration and/or autoclaving.

## Patentansprüche

1. Pharmazeutische Zusammensetzung zur parenteralen Verabreichung, die Paricalcitol oder einen Ester, Isomer, Solvat, Hydrat oder Polymorph davon, einen Alkohol, ausgewählt aus Ethanol, Propanol, Butanol oder Benzylalkohol, und eine wirksame Menge von Macrogol 15 Hydroxystearat, die die Zusammensetzung sowohl physikalisch als auch chemisch stabilisiert stellt niedrige Osmolalität bereit umfassend, wobei die Zusammensetzung frei von jeglichem zusätzlichen Konservierungsmittel ist.

2. Pharmazeutische Zusammensetzung nach Anspruch 1, wobei Macrogol-15-hydroxystearat in einer Menge zwischen 0,1% Gew./Vol. Bis 5% Gew./Vol. des Gesamtvolumens der Zusammensetzung vorliegt.

3. Pharmazeutische Zusammensetzung nach einem der vorhergehenden Ansprüche, in einer Phiole oder Ampulle oder einer vorgefüllten Spritze verpackt ist.

4. Verfahren zur Herstellung einer stabilen pharmazeutischen Zusammensetzung, die Paricalcitol oder einen Ester, Isomer, Solvat, Hydrat oder Polymorph davon umfassend, zur intravenösen Verabreichung, die die folgenden Stufen umfassend:
- Paricalcitol oder eines Esters, Isomers, Solvats, Hydrats oder Polymorphs davon in einer Lösung von Macrogol-15-Hydroxystearase mit einem Alkohol, ausgewählt aus Ethanol, Propanol, Butanol oder Benzylalkohol lösen;
- des Volumens mit Wasser zur Injektion einstellen, und optional
- durch Filtration und / oder Autoklavieren sterilisieren.

## Revendications

1. Une composition pharmaceutique pour administration parentérale comprenant le paricalcitol ou un ester, isomère, solvate, hydrate ou polymorphe de celui-ci, un alcool sélectionné parmi l'éthanol, le propanol, le butanol ou l'alcool benzylique et une quantité efficace de macrogol 15 hydroxystéarate qui stabilise la composition à la fois physiquement et chimiquement et fournit une faible osmolalité, où la composition est exempt de tout conservateur additionnel.

2. La composition pharmaceutique selon la revendication 1, dans laquelle le macrogol 15 hydroxystéarate est en quantité comprise entre 0.1% poids/volume et 5% poids/volume du volume total de la composition.

3. La composition pharmaceutique selon l'une quelconque des revendications précédentes conditionnée dans un flacon ou une ampoule ou une seringue pré-remplie.

4. Un procédé pour la préparation d'une composition pharmaceutique stable comprenant le paricalcitol ou un ester, isomère, solvate, hydrate ou polymorphe de celui-ci pour administration intraveineuse comprenant les étapes suivantes :
- dissoudre le paricalcitol ou un ester, isomère, solvate, hydrate ou polymorphe de celui-ci dans une solution de macrogol 15 hydroxystéarate avec un alcool sélectionné parmi l'éthanol, le propanol, le butanol ou l'alcool benzylique ;
- ajuster le volume avec de l'eau pour préparations injectables et, éventuellement
- stériliser par filtration et/ou autoclavage.
